# EUROPÄISCHE PATENTANMELDUNG

(11) **EP 1 982 653 A1**
(43) Veröffentlichungstag der Anmeldung: **22.10.2008**
(21) Anmeldenummer: 07007891.0
(22) Anmeldetag: 18.04.2007
(51) Int. Cl.: A61B 5/15

(54) **Stechgerät und Analysegerät**

(71) Anmelder: Roche Diagnostics GmbH, 68305 Mannheim (DE); F. Hoffmann-La Roche AG, 4070 Basel (CH)
(72) Erfinder: List, Hans, 64754 Hesseneck-Kalibach (DE); Haar, Hans-Peter, 69168 Wiesloch (DE)
(74) Vertreter: Jany, Peter

(57) **Zusammenfassung**

Die Erfindung betrifft ein Stechgerät (2) zum Erzeugen einer Einstichwunde in der Haut eines Menschen oder Tieres zum Gewinnen einer Probe einer Körperflüssigkeit für Analyse- oder Diagnosezwecke, insbesondere mit integrierten Disposables, die sowohl ein Stechelement (6) als auch eine zugehöriges Testelement umfassen. Um eine hohe Erfolgsrate bei dem Gewinnen einer Probe zu erzielen, wird vorgeschlagen, dass der Antrieb (5) derart ausgebildet ist, dass das Stechelement (6) oder das Testelement in eine Kontaktposition verfahrbar ist, in der es während einer Ausfahrdauer im Bereich der Körperteilanlageöffnung (13) in einer Position verbleibt, in der es von einem Benutzer des Stechgerätes (2) durch eine manuelle Positionierung des Stechgerätes (2) zum manuellen Aufnehmen einer Probe mit der Probenentnahmestelle in Kontakt bringbar ist wenn die bei einer Stechbewegung gewonnene Probemenge nicht zum Durchführen der Analyse oder Diagnose ausreicht.

## Beschreibung

Die Erfindung betrifft ein Stechgerät zum Erzeugen einer Einstichwunde in der Haut eines Menschen oder Tieres zum Gewinnen einer Probe einer Körperflüssigkeit mit einem ersten analytischen Verbrauchsmittel, das ein Stechelement umfasst, zum Durchführen einer Analyse eines medizinisch bedeutsamen Bestandteils der Probe mit einem zweiten analytischen Verbrauchsmittel, das ein Testelement umfasst, für Analyse- oder Diagnosezwecke anhand eines medizinisch bedeutsamen Bestandteils der Probe. Das Stechgerät umfasst ein Andruckteil zum Anpressen des Stechgeräts an einen Körperteil, in dem mit dem Stechelement an einer Probenentnahmestelle eine Einstichwunde erzeugt werden soll, aus der eine Probe entnommen werden soll, einen Antrieb zum Antreiben eines in das Stechgerät eingesetzten Stechelements für eine Stechbewegung des Stechelements entlang eines vorbestimmten Einstechweges, ein Gehäuse mit einer Körperteilanlageöffnung für den Körperteil, wobei das Stechelement in einer Ausgangsposition in dem Gehäuse angeordnet ist und in einer Stechposition an der Körperteilanlageöffnung mittels des Antriebs in den angelegten Körperteil einstechbar ist, und eine Kontrolleinrichtung zum Überprüfen einer gewonnenen Probe daraufhin, ob die gewonnene Probenmenge ausreicht, um mit einem Testelement die Analyse durchzuführen.

Die entnommene Körperflüssigkeit ist in der Regel Blut. In manchen Fällen handelt es sich aber auch um die Gewinnung einer Probe von interstitieller Flüssigkeit. Nachfolgend wird ohne Beschränkung der Allgemeinheit beispielhaft auf Blut, auch als Beispiel für andere aus einer Einstichwunde gewinnbare Körperflüssigkeiten, Bezug genommen.

Entsprechende Stechsysteme bestehen in der Regel aus zur einmaligen Verwendung vorgesehenen (disposiblen) Stechelementen zum Einstechen in die Haut und dem Stechgerät mit einem Stechelementantrieb für die Stechbewegung des Stechelements. Das Stechgerät eines solchen Stechsystems hat ein Andruckteil zum Anpressen an das Körperteil, in dem eine Einstichwunde erzeugt werden soll, und ein Auslösemittel, durch dessen Betätigung ein Benutzer eine Einstichbewegung eines Stechelements auslösen kann. Alternativ kann die Einstichbewegung auch automatisch durch das Andrücken des Stechgerätes auf die Haut ausgelöst werden. Das Stechelement ist beispielsweise eine Lanzette, eine Nadel oder ein Nadelelement. Derartige Stechelemente können beispielsweise in einem Magazin in ein Stechgerät eingesetzt werden.

Disposible Stechelemente werden seit langem benutzt, um für analytischdiagnostische Zwecke eine geringe Menge Blut aus einem Körperteil (meist aus einem Finger oder Ohrläppchen) zu entnehmen. In diesem Zusammenhang werden die Stechelemente üblicherweise als Lanzetten bezeichnet. Zum manuellen Einstechen vorgesehene Lanzetten werden in der Regel nur von medizinisch trainiertem Personal verwendet. Dennoch ist der Einstich mit erheblichem Schmerz verbunden.

Bereits seit langem werden auch Stechgeräte verwendet, die einen Stechelementantrieb enthalten. Häufig ist eine regelmäßige Überwachung bestimmter analytischer Werte des Blutes erforderlich. Stechgeräte werden insbesondere von Diabetikern verwendet, die mehrmals täglich mittels einer Blutzucker-Selbstkontrolle ihren Blutzuckerspiegel überprüfen müssen, um durch Anpassung von Insulininjektionen an den stark schwankenden Bedarf ihren Blutzuckerspiegel möglichst ständig innerhalb bestimmter Sollgrenzen zu halten. Entsprechend ist auch die Überprüfung von Blutgerinnungsparametern durch eine Patienten-Blutgerinnungs-Selbstkontrolle verbreitet. Das Stechgerät kann disposibel, d.h. zum nur einmaligen Gebrauch für das Gewinnen einer Probe bestimmt, mit einer fest integrierten Lanzette ausgebildet sein. In der Regel ist es jedoch mehrfach verwendbar und hat eine Halterung, mittels der jeweils eine Lanzette auswechselbar mit dem Stechantrieb gekoppelt werden kann. Da die Geräte und Lanzetten aneinander angepasste, von dem gleichen Hersteller gelieferte Elemente sind, werden sie als "Stechsystem" oder "Blutentnahmesystem" bezeichnet.

Als Antriebselement für den in einem Gehäuse des Stechgerätes angeordneten Lanzettenantrieb dient meist eine Feder oder ein elektromagnetischer Antrieb. Durch eine Lanzettenführung wird gewährleistet, dass die Stechbewegung auf einem vorbestimmten Einstichweg erfolgt. Dabei wird die Bewegung der Lanzette in Richtung auf die Hautoberfläche bis zu dem Umkehrpunkt (Vortriebsphase der Stechbewegung) angetrieben, beispielsweise mittels eines Federsystems oder eines elektromagnetischen Antriebs, und ein entsprechender Antrieb kann für die Rückzugsbewegung der Lanzette (Rückzugsphase der Stechbewegung) vorgesehen sein.

Bei den üblichen Konstruktionen haben die Stechgeräte an ihrem in Einstichrichtung des Stechelements vorderen Ende eine Austrittsöffnung, aus der die Spitze der Lanzette nur für kurze Zeit austritt, um eine Wunde in einem Körperteil zu erzeugen, gegen das das vordere Ende des Stechgerätes gedrückt wird. Die Stechtiefe ist dabei durch den Abstand in Einstichrichtung zwischen der Position, die die Lanzettenspitze am Umkehrpunkt der Lanzettenbewegung erreicht und der Ebene einer Hautkontaktfläche definiert, die die Austrittsöffnung ringförmig umgibt und im Moment des Einstichs an der Haut anliegt. Das vordere Ende des Stechgerätes mit der Hautkontaktfläche bildet also ein Stechtiefenreferenzelement, durch das gewährleistet wird, dass die Stechtiefe einem vorgegebenen Wert entspricht.

Um die Stechtiefe zu kontrollieren, ist es gebräuchlich, den Bewegungsweg der Lanzette in Einstichrichtung zu begrenzen, beispielsweise durch ein mit der Lanzette verbundenes Anschlagteil, das auf eine korrespondierende Anschlagfläche im Gehäuse des Stechgerätes auftrifft.

Derartige Blutentnahmesysteme müssen hohe Anforderungen erfüllen sind, wenn eine regelmäßige Überwachung bestimmter analytischer Werte des Blutes erforderlich ist. Dies gilt insbesondere für Diabetiker, die ihren Blutzuckerspiegel häufig kontrollieren sollten, um durch Anpassung von Insulininjektionen an den Bedarf (der abhängig von der Nahrungsaufnahme, der körperlichen Aktivität etc. stark schwankt) ihren Blutzuckerspiegel möglichst ständig innerhalb bestimmter Sollgrenzen zu halten. Durch umfangreiche wissenschaftliche Untersuchungen wurde bewiesen, dass mittels einer Intensivtherapie mit mindestens vier Blutanalysen pro Tag ein dramatischer Rückgang schwerster Spätschäden des Diabetes mellitus (beispielsweise einer Retinopathie mit resultierender Erblindung des Patienten) erreicht werden kann. Diese Intensivtherapie setzt voraus, dass die häufige Blutentnahme mit einem möglichst geringen Schmerz verbunden ist.

Ein ständiges Ziel in der Entwicklung von Stechsystemen besteht folglich darin, mit möglichst geringem Schmerz eine Einstichwunde zu erzeugen, aus der sich eine brauchbare Probe, d.h. eine ausreichende Menge einer Körperflüssigkeit, gewinnen lässt. Sowohl für das Schmerzempfinden als auch für die Probengewinnung ist die Einstichtiefe von großer Bedeutung. Generell gilt, dass das Schmerzempfinden mit zunehmender Einstichtiefe ebenso wie die Flüssigkeitsmenge, die sich aus der Einstichwunde gewinnen lässt, zunimmt. An Stechgeräte besteht deshalb die Anforderung, nur eine möglichst geringe Menge einer Blutprobe zu entnehmen, die für die Durchführung der Analyse ausreicht, und die Einstichtiefe einerseits so gering wie möglich, andererseits so tief wie nötig zu gestalten, und es gibt verschiedene Entwicklungen, um die Blutentnahme möglichst schmerzfrei zu gestalten.

In der Praxis wird von einem Blutentnahmesystem jedoch nicht nur erwartet, dass es der Anforderung an ein minimales Schmerzempfinden genügt, sondern gleichzeitig soll es eine einfache Bedienung aufweisen, eine kompakte schlanke Bauweise haben und eine einfache, kostengünstige Konstruktion ermöglichen. Aus diesen praktischen Anforderungen heraus wurden und werden Blutanalysegeräte entwickelt, die diesen teilweise gegenläufigen Anforderungen möglichst weitgehend genügen.

Da die Einstichtiefe auf einen möglichst geringen Wert eingestellt wird, kann es in der Praxis vorkommen, dass bei einem Einstich keine oder keine ausreichende Blutprobe gewonnen wird. Es werden daher im Stand der Technik Anstrengungen unternommen, um das Risiko eines erfolglosen Einstichs zu reduzieren. Beispielsweise sind Stechgeräte mit einem Drucksensor bekannt, bei denen automatisch eine Einstichbewegung ausgelöst wird, sobald auf dem Andruckteil ein Druck lastet, der einen vorgegebenen Mindestdruck übersteigt.

Während in Kliniken und bei niedergelassenen Ärzten oftmals durch eine Venenpunktion mehrere Milliliter Blut einer zu untersuchenden Person für die Analyse gewonnen werden, um damit eine Vielzahl von Labortests durchführen zu lassen, reichen für einzelne Analysen, die gezielt auf einen Parameter gerichtet sind, heutzutage oft wenige µl Blut aus. Die Gewinnung von geringen Probenmengen im Bereich weniger µl oder weniger zum Bestimmen analytischer Parameter ist insbesondere zur Messung des Blutglukosespiegels verbreitet, findet aber auch beispielsweise Anwendung bei der Bestimmung von Gerinnungsparametern, Triglyceriden, HBA 1c oder Lactat.

Solch geringe Blutmengen erfordern keine Venenpunktion sondern können mit Hilfe einer sterilen, scharfen Lanzette, die durch die Haut, z.B. in die Fingerbeere oder das Ohrläppchen der zu untersuchenden Person gestoßen wird, gewonnen werden. Insbesondere eignet sich diese Methode, wenn die Analyse der Blutprobe unmittelbar nach der Blutgewinnung durchgeführt werden kann.

Die zum Entnehmen von Körperflüssigkeit aus einem Körperteil durch Erzeugen einer kleinen Stichwunde verwendeten Lanzetten haben zumeist eine metallene Lanzettennadel, deren Spitze angeschliffen sein kann. Diese Lanzetten müssen bis zu ihrer Verwendung steril aufbewahrt werden und sollen nach ihrer Verwendung vorzugsweise so entsorgt werden, dass sie nicht zu Verletzungen führen können. Es wurden daher Blutentnahmesysteme vorgeschlagen, bei denen die Lanzetten in einem Lanzetten-Vorratsbehältnis, in dem eine Mehrzahl von Lanzetten zum Entnehmen aus dem Lanzetten-Vorratsbehältnis an einer Entnahmeposition vorrätig gehalten wird.

Eine mögliche Ausführungsform eines derartigen Lanzetten-Vorratsbehältnis ist ein Trommelmagazin, aus dem die Lanzetten einzeln entnehmbar sind, wobei die Lanzetten in Kammern in dem Trommelmagazin angeordnet sind, die jeweils einzeln verschlossen sind. Die gebrauchten Lanzetten werden dabei entweder außerhalb des Gerätes bzw. des Analysegerätes entsorgt oder können nach ihrem Gebrauch auch zum sicheren Entsorgen in das Lanzetten-Vorratsbehältnis zurückgeführt werden.

Die Erfindung betrifft auch ein Analysegerät zum Analysieren eines medizinisch bedeutsamen Bestandteils einer Körperflüssigkeit, beispielsweise Blut oder interstitielle Flüssigkeit. Dabei wird eine qualitative oder quantitative Analyse durchgeführt, also beispielsweise das Vorhandensein, das Nichtvorhandensein oder die Konzentration eines bestimmten Analyten in einer Probe bestimmt, wobei die Probe mit einem oben beschriebenen Stechgerät gewonnen wird, das in das Analysegerät integriert ist. Das Analysegerät umfasst also ein erfindungsgemäßes Stechgerät, wobei in das Analysegerät eine Analyseeinrichtung zum Durchführen einer Analyse oder Diagnose mittels der von dem Stechgerät gewonnenen Probe integriert ist. Die Erfindung bezieht sich insbesondere auf ein tragbares, netzunabhängig betreibbares und von einem Patienten bedienbares Analysegerät für die Patienten-Selbstkontrolle, den sogenannten Bereich des "Home-Monitoring", insbesondere ein Blutglukosemessgerät, ein Cholesterin-Messgerät oder ein Blutgerinnungsparameter-Messgerät.

Ein Blutglukosemessgerät ist ein Messgerät, mit dessen Hilfe der Blutzuckergehalt qualitativ oder quantitativ bestimmt werden kann. In der Regel wird hierzu in einem Körper eine Einstichwunde erzeugt, ein Bluttropfen entnommen, der Bluttropfen auf das Testelement aufgebracht und mit Hilfe des Testelements und des Blutglukosemessgeräts der Blutglukosegehalt in dem Tropfen bestimmt.

Die Erfindung bezieht sich also nicht nur auf Stechsysteme bzw. Stechgeräte, die ausschließlich dazu dienen, einen Bluttropfen für eine anschließende Analyse mit einem anderen Gerät zu gewinnen. Vielmehr richtet sie sich insbesondere auch auf sogenannte integrierte Systeme, mit denen nicht nur die Blutentnahme, sondern auch die Analyse durchgeführt wird, möglichst ohne zusätzliche Handhabungsschritte durch den Benutzer. Damit sind zusätzliche Anforderungen verbunden, die unter anderem aus der räumlichen Enge resultieren, wenn beide Funktionen in einem (aus Handhabungsgründen möglichst kleinen) Gerätegehäuse untergebracht werden müssen.

Derartige Analysegeräte umfassen ein Gerätegehäuse, eine in dem Gerätegehäuse angeordnete Messeinrichtung zum Durchführen der Analyse an einer mit dem Stechgerät gewonnenen Probe und einen Prozessor mit Software zum Verarbeiten der von der Messeinrichtung ermittelten Messwerte und zum Aufbereiten der Analysemessdaten aus den Messwerten, in der Regel unter Berücksichtigung von Kalibrationswerten.

Zur qualitativen und quantitativen Analyse von Bestandteilen einer flüssigen Probe, insbesondere einer Körperflüssigkeit von Menschen oder Tieren, werden in großem Umfang Testverfahren eingesetzt, die mit Testelementen arbeiten. Die Testelemente enthalten Reagenzien. Zur Durchführung einer Reaktion wird das Testelement mit der Probe in Kontakt gebracht. Die Reaktion von Probe und Reagenz führt zu einer für die Analyse charakteristischen Veränderung des Testelements, die mit Hilfe eines geeigneten Analysegerätes ausgewertet wird. Das Analysegerät ist in der Regel zum Auswerten eines ganz bestimmten Typs von Testelementen eines bestimmten Herstellers geeignet. Die Testelemente und das Analysegerät bilden wechselseitig aufeinander abgestimmte Bestandteile und werden insgesamt als Analysesystem bezeichnet.

Es sind zahlreiche unterschiedliche Testelement-Typen bekannt, die sich durch das Messprinzip und die verwendeten Reagenzien sowie durch ihren Aufbau unterscheiden. Die Verwendung von Magazinen für Testelemente und/oder Stechelemente ist in diesem Zusammenhang bekannt.

Hinsichtlich des Messprinzips sind kolorimetrische Analysesysteme besonders weit verbreitet. Bei ihnen führt die Reaktion der Probe mit den in dem Testelement enthaltenen Reagenzien zu einer Farbänderung, die visuell oder mittels einer photometrischen Messreinrichtung gemessen werden kann. Daneben haben elektrochemische Analysesysteme eine große Bedeutung erlangt, bei denen die Reaktion der Probe mit den Reagenzien des Testelements zu einer elektrisch messbaren Änderung (einer elektrischen Spannung oder eines elektrischen Stromes) führt, die mit einer entsprechenden Messelektronik gemessen wird. Derartige Analysesysteme werden auch als amperometrische Systeme bezeichnet.

Hinsichtlich des Aufbaus der Testelemente sind insbesondere streifenförmige Testelemente (sogenannte Teststreifen) gebräuchlich, die im wesentlichen aus einer länglichen Tragschicht aus Kunststoffmaterial und darauf angebrachten Testfeldern bestehen. Die Testfelder bestehen in der Regel aus einer oder mehreren Reagenzien enthaltenden Testschichten. Solche Teststreifen werden in großem Umfang insbesondere für Blut- und Urinuntersuchungen verwendet.

Bei einem zweiten Typ von Testelementen ist ein Testfeld ähnlich wie ein photographisches Diapositiv von einem Rahmen umgeben. Das Testfeld dieses Testelementtyps besteht in der Regel aus einer oder mehreren Testschichten, die von dem Rahmen gehalten werden und für kolorimetrische Tests geeignete Reagenzien enthalten. Nach Aufgabe der Probe auf das Testfeld und Ablauf der Testreaktion kann die Farbbildung beobachtet bzw. photometrisch vermessen werden.

Vor allem im Bereich des sogenannten "Home-Monitoring", also dort, wo medizinische Laien selbst einfache Analysen des Bluts durchführen, und dort insbesondere für die regelmäßige, mehrmals täglich durchzuführende Blutgewinnung durch Diabetiker für die Kontrolle der Blutglukosekonzentration, werden Lanzetten und dazu passende Geräte, sogenannte "Stechhilfen", angeboten, die eine möglichst schmerzarme und reproduzierbare Blutgewinnung ermöglichen, und es ist hier wichtig, dass eine einfache und zuverlässige Bedienung des Blutglukosemessgeräts möglich sowie eine informative und verlässliche Bestimmung und Darstellung der Messergebnisse vorhanden ist.

Die gebräuchlichen Analysegeräte sind sogenannte Stand-Alone-Messgeräte. Diese Geräte arbeiten autonom, selbständig und unabhängig. Sie haben also ein Display, eine Messeinrichtung, eine Stromversorgung und ein vollständiges User-Interface, das z.B. eine Tastatur, eine Anzeige, einen Signalgeber oder eine Benutzerführung umfassen kann. Der Anwendungszweck und die Eigenschaften derartiger Geräte sind, bis auf gelegentliche Anpassungen der Firmware, festgelegt.

So geht beispielsweise ein bekanntes Gerätekonzept für Blutanalysegeräte davon aus, dass "integrierte Disposables" verwendet werden, bei denen es sich um eine integrierte Kombination von jeweils einem analytischen Verbrauchsmittel (Disposable) in Form eines Stechelements (Nadelelement zum Durchführen des Einstichs) und einem analytischen Verbrauchsmittel (Disposable) in Form eines Testelements (z.B. Testchemie zum Durchführen der Analyse oder Diagnose) handelt. Dabei ist also jeweils für ein Testelement ein Stechelement vorgesehen bzw. vorhanden, das in das Testelement integriert ist. Bei einem integrierten Disposable sind also das erste und das zweite analytische Verbrauchsmittel in einem analytischen Verbrauchsmittel integriert, das sowohl ein Stechelement zum Durchführen des Einstichvorgangs für das Gewinnen der Probe einer Körperflüssigkeit als auch ein Testelement zum Durchführen der Analyse eines medizinisch bedeutsamen Bestandteils der Probe umfasst. Je nach Schwerpunkt der Sichtweise spricht man hierbei auch von einem Stechelement mit integriertem Testelement oder von einem Testelement mit integriertem Stechelement.

Die Übertragung des mit dem Stechelement gewonnen Bluttropfens zu einem Analysesensor bzw. Testelement erfolgt beispielsweise mit Hilfe eines in dem Gerätegehäuse verlaufenden Kapillarkanals. Dies ist ein Beispiel für ein Stechsystem, dessen Nadelelement einen Kapillarkanal aufweist, durch den eine Körperflüssigkeit aus der Haut in das Innere der Stecheinheit transportiert werden kann.

Ein weiteres Beispiel eines solchen Stechsystems ist in der US-Patentanmeldung US 2003/0018282 A1 beschrieben. Dabei umfasst die Stecheinheit nicht nur die in die Haut einzustechende Nadel mit einem zum Transport der Probe geeigneten Kapillarkanal, sondern auch einen Reagenzien enthaltenden Nachweisbereich. Eine solche Stecheinheit, die gleichzeitig einen (beispielsweise als kapillaraktive Saugschicht und/oder Hohlkammer ausgebildeten) Aufnahmebereich für die Probe hat und vorzugsweise auch die für die Analyse erforderlichen Reagenzien enthält, wird auch als "Mikrosampler" bezeichnet. Hinsichtlich näherer Einzelheiten über Mikrosampler wird auf die erwähnte US-Patentanmeldung und die darin zitierten Dokumente, insbesondere das US-Patent 5,801,057 verwiesen. Abgesehen von den hier beschriebenen Besonderheiten können im Rahmen der vorliegenden Erfindung Mikrosampler unterschiedlicher Konstruktionen eingesetzt werden.

Die vorliegende Erfindung richtet sich generell auf Stechsysteme unterschiedlicher Typen, wobei das Nadelelement, das in die Haut eingestochen wird, als massive Nadel (wie bei den erwähnten Lanzetten) oder als Kapillarnadel (mit offener Kapillare oder als geschlossene Hohlnadel) ausgebildet sein kann. Soweit auf Lanzetten oder andere speziellere Ausführungsformen Bezug genommen wird, geschieht dies beispielhaft und ohne Beschränkung der Allgemeinheit. Die erläuterten Sachverhalte gelten grundsätzlich auch für andere Ausgestaltungen.

Die Erfindung eignet sich insbesondere für integrierte Systeme, bei denen die Funktionen der Blutgewinnung und der Analyse in einem Gerät vereinigt sind. Im Falle eines Mikrosamplers erfolgt diese Integration vorzugsweise dadurch, dass in dessen Probenaufnahmebereich die für die Analyse erforderlichen Reagenzien und sonstigen Komponenten vorhanden sind. Insoweit unterscheiden sich für die Erfindung geeignete Mikrosampler nicht von bekannten Systemen. Auch die Stechelemente ohne Kapillarkanal können in Verbindung mit der Erfindung in vorteilhafter Weise in integrierten Systemen verwendet werden, wobei in diesem Fall das Stechelement nach dem Stechvorgang rasch zurückgezogen wird, so dass die aus der Haut austretende Probenflüssigkeit in einen Kapillarkanal eines Analyseelementes eindringen kann, das zu diesem Zweck innerhalb des Stechgerätes in Kontakt mit der aus der Haut austretenden Probenflüssigkeit gebracht wird.

Es können auch integrierte Disposables verwendet werden. Integrierte Disposables sind Verbrauchselemente, die sich dadurch auszeichnen, das sie sowohl ein Stechelement zum Durchführen des Einstichs für das Gewinnen einer Körperflüssigkeitsprobe, beispielsweise einer Blutprobe, als auch ein Testelement, z.B. eine Testchemie zum Durchführen der Analyse eines medizinisch bedeutsamen Bestandteils der Probe beinhalten. Solche Disposables werden häufig in geeigneten Magazinen, die z.B. die Form eines Streifens haben, verwendet.

Die Erfindung bezieht also in Bezug auf Analysegeräte allgemein auf jede Kombination analytischer Verbrauchsmittel in Form von Stechelementen, Testelementen und integrierten Disposables. Der Bluttransport bzw. der Transport mittels des Einstichs mit dem Stechelement gewonnener Körperflüssigkeit von der Einstichstelle zu dem Testelement kann auf jede bekannte Weise erfolgen, beispielsweise durch eine Aufnahme mit dem Stechelement selbst mit anschließendem Transport von dem Stechelement zu dem Testelement, z.B. in einer Kapillare, oder durch eine Aufnahme mit dem Testelement direkt, wobei nach dem Stechen mit dem Stechelement mit einem geeigneten Antrieb das Testelement mit der Einstichstelle in Kontakt gebracht wird.

In der Patentliteratur gibt es eine Reihe von integrierten Tests zur Blutanalyse. Sie beschreiben aber meist nur den Testaufbau (US 6607658 und EP1402812 als Beispiele für Testelemente mit integrierter Lanzette, oder US 2002/0168290 als Beispiel für Sampler mit integrierter Testchemie bzw. integriertem Sensor). Einige Veröffentlichungen befassen sich mit Antrieben für das Stechelement, was von einfachen ballistischen Federmechaniken (US 2006/0178600) bis zum elektrischen Direktantrieb reicht. Darüber hinaus befassen sich einige Veröffentlichungen mit Methoden, das Austreten von Blut durch bestimmte Bewegungsprofile zu unterstützen (z.B. US 7025774).

Integrierte Disposables sind beispielsweise auch in den Dokumenten US 6,607,658 oder US 2002/0168290 beschrieben. Nach einem Stechvorgang durch ein Nadelelement erfolgt anschließend automatisch eine Probenaufnahme, die entweder direkt durch das Testelement erfolgen kann, siehe z.B. US 6,607,658 oder durch eine Kapillarstruktur der Nadel, wobei das Blut anschließend zum Testfeld hingeleitet wird. Ein integriertes System mit entsprechenden Antriebseinheiten beschreibt z.B. die US 7,025,774.

Keine Veröffentlichung geht dabei aber darauf ein, dass im Alltag die Situation nicht selten ist, dass nach einem Einstich in die Haut keine oder keine ausreichende Probe gewonnen werden kann, sei es dass die Handhabung fehlerhaft war, sei es, dass die korrekte Tiefeneinstellung für den Stich noch nicht ermittelt wurde.

Bevor das Blut jedoch durch das Disposable aufgenommen werden kann, muss zuvor eine automatische Blutexpression erfolgen, die meist durch einen sogenannten Fingerkonus oder Ring erfolgt, der entsprechend Druck auf die Fingerkuppe ausübt, so dass der Blutexpressionsschritt unterstützt wird. Eine Blutaufnahme kann so bereits während des Stechvorgangs z.B. durch das Nadelelement unterhalb der Haut erfolgen, oder anschließend von der Hautoberfläche. In der Praxis zeigt sich, dass der Erfolg des Blutexpressionsschrittes sowie der Blutaufnahme häufig von vielen weiteren Faktoren (Elastizität der Haut, Hauttemperatur etc.) abhängt, so dass die zur Zeit zu erreichende "Erfolgsrate" des Expressions- bzw. Blutsammelvorgangs nicht bei 100% liegt. Da üblicherweise die Disposables nur zur Einmalverwendung vorgesehen sind, wird das Disposable bei nicht erfolgreichem Sammelvorgang also unbenutzt verworfen.

Bei derartigen integrierten oder hochintegrierten Messgeräten, insbesondere bei solchen für den mobilen Gebrauch wie beim Home-Monitoring, stellen sich somit verschiedene Probleme:
- Nach dem Stand der Technik wird bei jeder Blutentnahme eine frische Lanzette verwendet. Daraus resultiert ein Lanzettenmagazin, das mehr Volumen in dem Blutanalysegerät beansprucht, als das Magazin für die Testelemente. Zur Verkleinerung des Blutanalysegerätes ist es wünschenswert, das Volumen des Lanzetten-Vorratsbehältnisses zu reduzieren.
- Im Stand der Technik wird davon ausgegangen, dass das integrierte Disposable, d.h. das kombinierte Stech- und Testelement zum einmaligen Gebrauch bestimmt ist und nach dem Abschluss eines Benutzungszykluses verworfen wird. Verworfen wird dann auch ein an sich ungebrauchter Test, wenn die Blutgewinnung nicht funktioniert hat. Für einen neuen Versuch muss ein neues Disposable verwendet werden.
- Für die Benutzer integrierter Disposables ist wegen der Kosten dieser Verbrauchsmaterialien, die höher sind als bei einfachen Stech- oder Testelementen, sehr wichtig, dass die Probenanalyse mit einer sehr hohen Erfolgsrate durchgeführt wird, so dass keine Disposables verschwendet werden. Diese Erfolgsrate muss deutlich über 90% liegen und in der Praxis nahezu 100% betragen, damit ein entsprechendes System von den Benutzern akzeptiert wird, insbesondere weil sie in zunehmendem Maß die Kosten für die Verbrauchsmaterialien selbst tragen müssen. Zur Erzielung einer hohen Erfolgsrate ist nach dem Stand der Technik ein großer technischer Aufwand erforderlich.

Der Erfindung liegt die Aufgabe zugrunde, ein Stechgerät bzw. ein Analysegerät zu schaffen, bei dem mit geringem technischem Aufwand einer sehr hohe Erfolgsrate beim Gewinnen einer Probe einer Körperflüssigkeit, insbesondere mit integrierten Disposables, erzielt wird.

Diese Aufgabe wird erfindungsgemäß durch ein Stechgerät bzw. ein Verfahren oder ein Analysegerät mit den Merkmalen der beigefügten unabhängigen Patentansprüche gelöst. Bevorzugte Ausgestaltungen und Weiterbildungen der Erfindung ergeben sich aus den abhängigen Patentansprüchen und der nachfolgenden Beschreibung mit zugehöriger Zeichnung.

Ein erfindungsgemäßes Stechgerät zum Erzeugen einer Einstichwunde in der Haut eines Menschen oder Tieres zum Gewinnen einer Probe einer Körperflüssigkeit mit einem ersten analytischen Verbrauchsmittel, das ein Stechelement umfasst, zum Durchführen einer Analyse eines medizinisch bedeutsamen Bestandteils der Probe mit einem zweiten analytischen Verbrauchsmittel, das ein Testelement umfasst, wobei das Stechgerät ein Andruckteil zum Anpressen des Stechgeräts an einen Körperteil, in dem mit dem Stechelement (6) an einer Probenentnahmestelle eine Einstichwunde erzeugt werden soll, aus der eine Probe entnommen werden soll, einen Antrieb zum Antreiben eines in das Stechgerät eingesetzten Stechelements für eine Stechbewegung des Stechelements entlang eines vorbestimmten Einstechweges, ein Gehäuse mit einer Körperteilanlageöffnung für den Körperteil, wobei das Stechelement in einer Ausgangsposition in dem Gehäuse angeordnet ist und in einer Stechposition an der Körperteilanlageöffnung mittels des Antriebs in den angelegten Körperteil einstechbar ist, und eine Kontrolleinrichtung zum Überprüfen einer gewonnenen Probe daraufhin, ob die gewonnene Probenmenge ausreicht, um mit einem Testelement die Analyse durchzuführen, umfasst, weist also die Besonderheit auf, dass der Antrieb derart ausgebildet ist, dass mindestens eines der analytischen Verbrauchsmittel in eine Kontaktposition verfahrbar ist, in der es während einer Ausfahrdauer im Bereich der Körperteilanlageöffnung in einer Position verbleibt, in der es von einem Benutzer des Stechgerätes durch eine manuelle Positionierung des Stechgerätes zum manuellen Aufnehmen einer Probe mit der Probenentnahmestelle in Kontakt bringbar ist, wenn gemäß dem Ergebnis einer mit der Kontrolleinrichtung durchgeführten Überprüfung der gewonnenen Probenmenge die bei einer vorausgegangenen Stechbewegung gewonnene Probenmenge nicht zum Durchführen der Analyse oder Diagnose ausreicht.

Durch ein erfindungsgemäßes Stechgerät mit einer Antriebseinheit, die zum einen den Stechvorgang ausführen kann, sowie zum anderen einen Verfahrschritt des Disposables durchführt, wobei diese Funktionen auch durch zwei getrennte Antriebe realisiert werden können, bei dem eine händige Blutaufgabe möglich ist, lassen sich die Nachteile der bekannten Geräte beheben, indem ein (erneutes) Wiederverwenden das Stechelements bzw. bei einem System für integrierte Disposables des integrierten Disposables ermöglicht wird, so dass entweder der Stechvorgang wiederholt werden kann, oder das Disposable (erneut) aus dem System gefahren wird, so dass eine händige Blutaufgabe durch den Benutzer möglich ist, nachdem manuell durch das sogenannte "Melken" des Fingers Blut gewonnen wurde.

Der erfindungsgemäße Bewegungsablauf unterscheidet sich also vom Stand der Technik dadurch, dass mindestens eines der analytischen Verbrauchsmittel (Stechelement oder Testelement) mittels des Antriebs (des Stechelements oder des Testelements) in eine Kontaktposition verfahren wird, in der es nicht nur ganz kurz für den Einstich verbleibt, sondern während einer Ausfahrdauer im Bereich der Körperteilanlageöffnung in einer Position verbleibt, in der es von einem Benutzer des Stechgerätes durch eine manuelle Positionierung des Stechgerätes zum manuellen Aufnehmen einer Probe mit der Probenentnahmestelle in Kontakt gebracht werden kann, wenn gemäß dem Ergebnis einer mit der Kontrolleinrichtung durchgeführten Überprüfung der gewonnenen Probenmenge die bei einer vorausgegangenen Stechbewegung gewonnene Probenmenge nicht zum Durchführen der Analyse oder Diagnose ausreicht.

Die Ausfahrdauer des analytischen Verbrauchsmittels aus dem Gerät in die Kontaktposition im Bereich der Körperteilanlageöffnung kann fest vorgegeben sein, beispielsweise mehrere Sekunden bis einige Minuten, nach deren Ablauf das analytische Verbrauchsmittel automatisch von dem Antrieb in das Gerät zurückgezogen oder ausgeworfen wird. Das Ende der Ausfahrdauer kann dabei dem Benutzer durch vorausgehende Signale angekündigt werden. In anderen Ausführungsformen kann die Ausfahrdauer aber auch erst durch ein Betätigung eines Bedienelements durch den Benutzer beendet werden.

Bei dem in der Kontaktposition des analytischen Verbrauchsmittels manuell von dem Benutzer hergestellten Kontakt zwischen Körperteil bzw. Probenentnahmestelle und dem analytischen Verbrauchsmittel, das die Körperflüssigkeit aufnimmt, kann der Körperteil in derselben Position wie bei dem Einstechen an der Körperteilanlageöffnung des Gerätes anliegen oder sich vorzugsweise auch in einem Abstand dazu befinden, damit der Benutzer die Probenentnahmestelle besser einsehen und den Kontakt herstellen kann.

Mit der erfindungsgemäßen Erfolgskontrolle ist es also möglich, für den Fall, dass das erste Stechen nicht zu einer erfolgreichen Blutentnahme geführt hat, dies zu erkennen und die zuvor gestochene Fingerbeere, die noch die Einstichwunde aufweist, zu kneten und zu massieren ("melken"), um die Durchblutung zu fördern und dadurch einen Blutaustritt zu erzielen. Das auf diese Weise gewonnene Blut wird dann händig durch den Benutzer an der Auftragstelle des analytischen Verbrauchsmittels zur Analyse aufgegeben.

Diese Vorgehensweise ist in der Regel sogar vorteilhafter als ein zweites Stechen, weil ein zweites Stechen die Erfolgsquote für das Gewinnen einer ausreichenden Blutmengenprobe nicht unbedingt erhöhen würde, beispielsweise weil an der zuvor gestochenen Stelle wegen einer geringen Durchblutung wenig Blut vorhanden ist. Das Erkennen eines beim ersten Stechen auftretenden Fehlers und das nachfolgende händige Auftragen der Blutprobe mit vorausgehendem Kneten der Einstichstelle weist den in der Praxis sehr großen Vorteil auf, dass auch bei ungünstigen Bedingungen der Probeentnahmestelle mit geringem technischen Aufwand eine sehr hohe Erfolgsrate bei der Verwendung eines Stechelements, insbesondere eines integrierten Disposables, von über 95% und somit eine große Akzeptanz für den Benutzer erzielt werden kann.

Gemäß einer anderen Ausführungsform ist es aber auch möglich, dass nach dem ersten Stich mit einem Stechelement eine Kontrolle des Blutgewinnungserfolges zwischengeschaltet wird, wonach ggf. die Stecheinrichtung erneut in Bereitschaft versetzt wird, damit der Anwender u.U. nach einer Korrektur der Stechtiefeneinstellung erneut einen Stich ausführen kann, ohne das Stechelement oder Disposable vorher austauschen zu müssen.

Nach einem ersten vorteilhaften Merkmal kann vorgesehen sein, dass die Kontrolleinrichtung zum visuellen Kontrollieren der Probenmenge durch den Benutzer des Gerätes ausgebildet ist. Die Kontrolle der ausreichenden Probenaufnahme kann beispielsweise im Falle von Systemen mit Testelementen mit integrierter Lanzette durch den Anwender visuell erfolgen. Da keine automatische Blutgewinnung erfolgt, sondern von Hand gemolken werden muss, fällt das besonders leicht. Je nach Ausführungsform des Gerätes kann einfach erneut der Stechapparat gespannt werden oder ein Menüpunkt zur Stichwiederholung wird aufgerufen, wonach der Stechantrieb wieder in Bereitschaft tritt. Diese Funktion kann im Anwendungsgerät (Stechgerät oder Analysegerät) vorgesehen werden, was je nach Funktionsweise leicht möglich ist.

In anderen Ausführungsformen kann auch vorgesehen sein, dass die Kontrolleinrichtung eine automatische Probenmengenerkennungseinrichtung oder Unterdosiererkennungseinrichtung umfasst. Bei einer automatischen Bluterkennung, gemäß der festgestellt wird, dass nicht ausreichend Blut auf das Testfeld gelangt ist, kann automatisch das Disposable zur händigen Blutaufgabe verfahren werden. Eine automatische Bluterkennung bzw. Unterdosierungserkennung ist im Stand der Technik für Teststreifen hinreichend bekannt. Beispielsweise beschreibt das Dokument US 6,055,060 eine Unterdosierungserkennung. Vorteilhafterweise ist darüber hinaus das System in der Lage, zunächst einen wiederholten Stechvorgang auszuführen, bevor eine händige Blutaufgabe durch den Benutzer möglich ist.

Bei Systemen mit automatischer Blutgewinnung und ebenso automatischem Transfer der gewonnenen Probe auf die Testchemie kann beispielsweise die Benetzungskontrolle der Testchemie bzw. die Testauswertung dazu herangezogen werden, den Sammelerfolg festzustellen und ggf. automatisch mit demselben Stechelement bzw. Disposable erneut Stichbereitschaft herzustellen. Hierbei eignen sich kurvengesteuerte Systeme nach dem OWADAC-Prinzip (OWADAC= one ay alternating drive and cocking) besonders gut, aber auch ballistische Systeme lassen sich ohne großen Aufwand so gestalten, dass ohne Austausch des Stechelements bzw. Disposables der Startzustand wiederhergestellt werden kann.

Selbst wenn im Falle von Samplern der Erfolg der Blutgewinnung erst bei der Übertragung auf die Testchemie festgestellt werden kann, kann in den meisten Ausführungsformen von Disposables (Stechelemente oder integrierte Disposables) und Analysegeräten die Testsequenz um einige Schritte zurückgefahren werden, so dass mit dem selben Disposable ein neuer Versuch gestartet werden kann. Sogar bei vielen Systemen mit Magazinierung von Testelementen steht einem zweiten Versuch der Blutgewinnung mit demselben Disposable nichts im Wege. Es ist lediglich darauf zu achten, dass für die verschiedenen Ablaufschritte keine irreversiblen Vorgänge herangezogen werden wie z.B. das Weiterspulen von Bändern, wenn aus Platz- und Kostengründen keine Rückspulfunktion vorgesehen ist, oder das Umbiegen von Blechteilen für die Blutübertragung wie bei einigen Varianten von Samplern.

Im Zusammenhang von "GAM" (= get & measure) mit den Disposables, bei dem Stechen und Messen vollständig in ein Gerät integriert ist, ergibt sich zusätzlich noch eine Optimierung, die den Erfolg für den Anwender, zu einem Analysewert, beispielsweise einem Blutglukosewert zu gelangen, noch erhöht.

Die Kontrolleinrichtung kann eine Signaleinheit zum Signalisieren einer für die Durchführung der Analyse oder Diagnose zu geringen Probenmenge an den Benutzer umfassen. Die Signaleinrichtung kann ein optisches, akustisches, haptisches oder sonstiges Signal abgeben. Durch diese kann dem Benutzer beispielsweise signalisiert werden, dass er sich entscheiden muss, ob er mit dem Stechelement bzw. dem integrierten Disposable einen weiteren Einstichversuch an derselben oder einer anderen Probenentnahmestelle durchführen möchte oder lieber einen händigen Probenauftragsschritt durchführen möchte, wobei der Benutzer seine Entscheidung durch Betätigen eines Bedienelement eingeben kann. Demgemäß kann es vorteilhaft sein, wenn das Stechelement zum Durchführen eines weiteren Einstichvorgangs mit dem Stechelement in die Ausgangs- oder eine Bereitschaftsposition verfahrbar ist, wenn bei der Verwendung des Stechelements zum Entnehmen einer Probe erstmals eine für die Durchführung der Analyse oder Diagnose zu geringe Probenmenge erkannt wird. Der weitere Stechvorgang kann vorteilhafterweise mit einer vergrößerten Einstichtiefe durchführbar sein. In anderen Ausführungsformen kann dem Benutzer durch die Signaleinrichtung auch direkt signalisiert werden, dass als nächstes eine händige Probenaufgabe mit dem Stechelement bzw. integrierten Disposable durchzuführen ist.

Allgemein kann es vorteilhaft sein, wenn das Stechelement in die Kontaktposition verfahrbar ist, wenn bei der Verwendung des Stechelements zum Entnehmen einer Probe erstmals eine für die Durchführung der Analyse oder Diagnose zu geringe Probenmenge erkannt wird. Vorteilhafte Ausführungsformen in Verbindung mit einer automatischen Kontrolleinrichtung können darin bestehen, dass das Stechelement automatisch in die Kontaktposition verfahren wird, wenn die Kontrolleinrichtung eine für die Durchführung der Analyse oder Diagnose zu geringe Probenmenge erkennt.

Weitere Einzelheiten und Vorteile der Erfindung werden anhand eines Ausführungsbeispiels unter Bezugnahme auf die beigefügten Zeichnungen erläutert. Die dabei beschriebenen Merkmale können einzeln und in Kombination verwendet werden, um bevorzugte Ausgestaltungen der Erfindung zu schaffen. Gleiche oder einander entsprechende Bauteile sind dabei mit übereinstimmenden Bezugszeichen gekennzeichnet.

Es zeigen:
- Fig. 1: eine Ansicht eines Analysegerätes mit einem integriertem erfindungsgemäßen Stechgerät an einem Körperteil;
- Fig. 2: einen ersten Einstechvorgang mit einem Stechgerät;
- Fig. 3: einen Rückzugsvorgang zu Fig. 2;
- Fig. 4: einen zweiten Einstechvorgang mit vergrößerter Einstichtiefe;
- Fig. 5: einen Rückzugsvorgang Fig. 4;
- Fig. 6: einen ersten Teilschritt eines manuellen Blutauftrags auf ein Stechelement; und
- Fig. 7: einen zweiten Teilschritt zu Fig. 6.

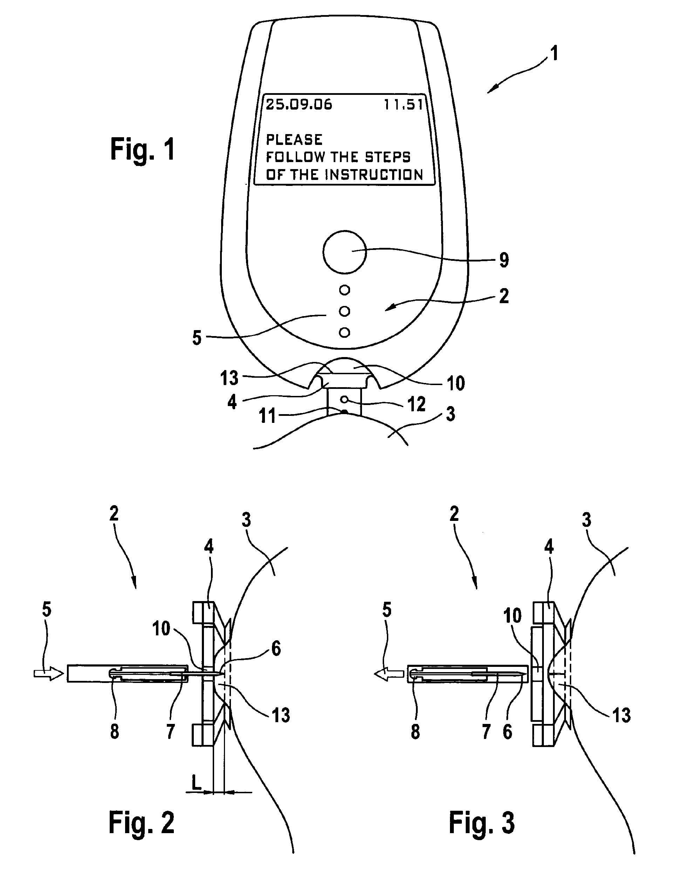

Die Fig. 1 zeigt eine Ansicht eines Ausführungsbeispiels eines Analysegeräts 1 mit einem integrierten Stechgerät 2 zum Erzeugen einer Einstichwunde in einer Fingerkuppe 3 zum Gewinnen und Analysieren einer Blutprobe aus der Fingerkuppe 3. Hierzu umfasst es ein Andruckteil 4 zum Anpressen an die Fingerkuppe 3, aus der die Probe entnommen werden soll, und einen Antrieb 5 zum Antreiben eines Stechelements 6 für eine Stechbewegung. Das Andruckteil 4 ist als Andruckkonus ausgebildet, der in der angepressten Fingerkuppe 3 einen erhöhten Körperflüssigkeitsdruck erzeugt und somit den Austritt von Körperflüssigkeit aus einer mit einem in das Stechgerät 2 eingesetzten Stechelement 6 erzeugten Einstichwunde begünstigt.

Bei dem in Fig. 1 schematisch dargestellten Analysegerät 1 handelt es sich um ein integriertes System zur Gewinnung und Analyse einer Körperflüssigkeitsprobe. Das Stechelement 6 enthält deshalb einen Kapillarkanal 7, der in eine Analysezone 8 mündet, die mit Testchemikalien behandelt ist und deshalb eine von der Analytkonzentration abhängige Farbänderung erfährt.

Zur Analyse wird die Analysezone 8 mit einer Lichtquelle bestrahlt und reflektierte Strahlung von einem Detektor detektiert. Die Lichtquelle wird von einer Steuereinheit gesteuert und das Signal des Detektors von einer Auswerteeinheit ausgewertet. Vorzugsweise regelt die Auswerteeinheit auch die Steuereinheit. Die Auswerteeinheit führte eine Auswertung des Detektorsignals durch, um die Konzentration des in der Probe der Körperflüssigkeit enthaltenen Analyten zu ermitteln. Das Analyseergebnis wird mit einer Ausgabeeinheit, beispielsweise einer Flüssigkristallanzeige ausgegeben.

Einen besonders hohen Benutzerkomfort bieten somit Stechgeräte 2, mit denen nicht nur eine Einstichwunde erzeugt wird, sondern die zusätzlich eine Messeinrichtung zum Untersuchen einer gewonnenen Körperflüssigkeitsprobe umfassen. Geeignete Messeinrichtungen, beispielsweise zur photometrischen oder elektrochemischen Untersuchung, sind in handelsüblichen Analysehandgeräten 1 zum Bestimmen des Blutglukosegehalts enthalten. Beispielsweise können eine Probenaufnahmeeinheit und ein Testfeld zur elektrochemischen oder photometrischen Bestimmung einer Analytkonzentration in das Stechelement 6 integriert sein. Geeignete Stechelemente, die einen Kapillarkanal aufweisen, in den Körperflüssigkeit während einer Sammelphase eindringen und zu dem Testfeld gelangen kann, sind im Stand der Technik, beispielsweise aus der US 2003/0171699 A1, bekannt. Bei derartigen Stechgeräten macht die Probenaufnahme für den Benutzer keine zusätzlichen Handhabungsschritte erforderlich. Dies ist insbesondere für Benutzer, deren motorische Beweglichkeit durch Alter oder Krankheit eingeschränkt ist, ein wichtiger Vorteil.

Das dargestellte Analysegerät 1 weist ein Auslösemittel auf, durch dessen Betätigung ein Benutzer nach dem Anpressen oder durch Anpressen des Andruckteils 4 eine Einstichbewegung auslösen kann. Bei dem dargestellten Ausführungsbeispiel ist das Auslösemittel als Knopf 9 ausgebildet. Dabei kann auch eine Sicherungseinrichtung vorgesehen sein, mit der das Stechgerät 2 aus einem Sperrzustand in einem Auslösezustand versetzt wird.

Die Figuren 2 bis 7 zeigen die für die erfindungsgemäße Funktion wesentlichen Komponenten des Stechgeräts 2 in dem Analysegerät 1 von Fig. 1. Die Figuren 2 und 3 zeigen einen ersten Schritt, nämlich in Fig. 2 einen Einstechvorgang mit dem Stechgerät 2 mit einer Einstechtiefe L von beispielsweise 1,0 mm. Die Fingerkuppe 3 wird zum Durchführen des Einstichs in der Körperteilanlageöffnung 13 an das Gerät 1 bzw. das Andruckteil 4 angepresst. Die Fig. 3 zeigt die Position des Stechelements 6 nach seinem vollständigen Zurückziehen in das Stechgerät 2, und in dieser Position wird visuell oder automatisch geprüft, ob bei dem Einstechvorgang gemäß Fig. 2 eine ausreichende Probenmenge gewonnen wurde.

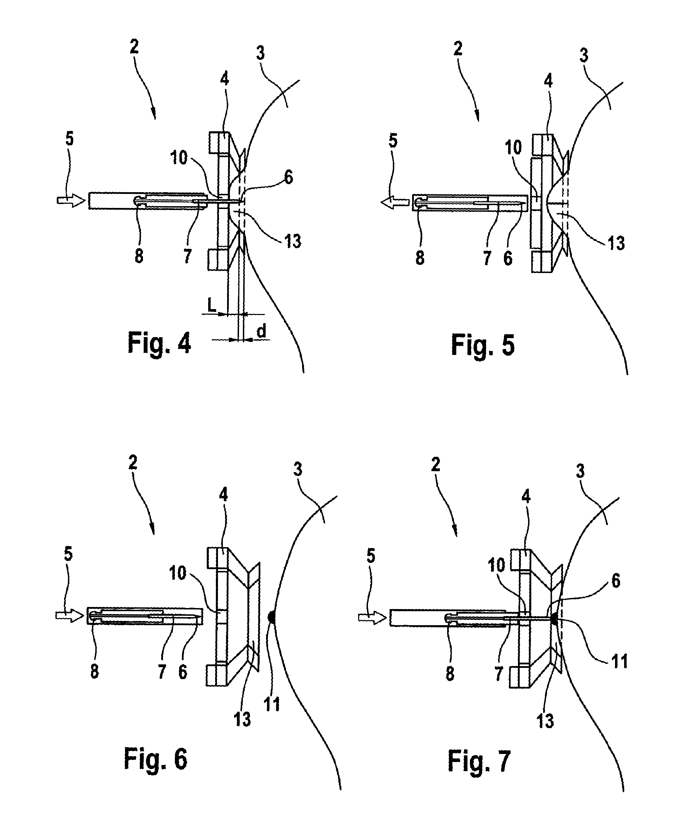

Ist dies nicht der Fall, kann der in den Figuren 4 und 5 dargestellte zweite Schritt eines weiteren Einstechvorgangs manuell oder automatisch ausgelöst werden. Dabei zeigt Fig. 4 einen Einstechvorgang, der mit einer erhöhten Einstechtiefe, beispielsweise mit einer Einstechtiefenzugabe d von 0,5 mm durchgeführt werden kann, und Fig. 5 zeigt das Stechelement 6 in derselben Position wie in Fig. 3, wobei wiederum geprüft wird, ob eine ausreichende Menge einer Probe gewonnen wurde.

Wenn dies nicht der Fall ist, erfolgt der in den Figuren 6 und 7 dargestellte dritte Schritt, wobei dieser Schritt auch unmittelbar nach dem ersten Schritt (Figuren 2 und 3) unter Auslassung des zweiten Schrittes (Figuren 4 und 5) durchgeführt werden kann, wenn gemäß Fig. 3 festgestellt wird, dass keine ausreichende Probenmenge gewonnen wurde. Die Fig. 6 zeigt das Stechelement 6 noch in der in das Stechgerät 2 zurückgezogenen Ausgangsposition entsprechend zu den Figuren 2 und 4, aus der es gemäß Fig. 7 in eine Kontaktposition verfahren wird, in der es aus der Austrittsöffnung 10 des Stechgerätes 2 im Bereich der Körperteilanlageöffnung 13 derart während einer gewissen Ausfahrdauer herausragt, dass es von einem Benutzer des Gerätes durch eine manuelle Positionierung des Stechgerätes 2 mit der Probenentnahmestelle in Kontakt gebracht werden kann. Dabei wird der Benutzer vorteilhafterweise das Gebiet um die Probenentnahmestelle zuvor kneten oder massieren, um das Austreten eines Bluttropfens 11 zu fördern, der dann von der Spitze des aus dem Stechgerät 2 mittels des Antriebs herausgefahrenen Stechelements 6 in Kontakt gebracht werden kann, wodurch mit dem Stechelement 6 die Probe aus dem Bluttropfen 11 entnommen wird.

Das manuelle Aufnehmen eines Bluttropfens 11 mit dem Stechelement 6 kann durch eine schrittweise Benutzerführung, die in einer Anzeige des Analysegerätes 1 erscheint, geführt werden. Zum Erleichtern der manuellen Aufnahme der Probe mit dem Stechgerät 2 kann es vorteilhaft sein, wenn die Probenaufnahmestelle mittels einer in das Stechgerät 2 bzw. das Analysegerät 1 integrierten Beleuchtung beleuchtet wird. Die Fig. 1 zeigt diese Beleuchtung 12 in einer Position des Analysegeräts 1 gemäß Fig. 6.

In abgewandelten Ausführungsformen kann die manuelle Probenaufnahme gemäß dem dritten Schritt auch dadurch erfolgen, dass nicht das Stechelement 6, sondern das Testelement, mit dem die Analyse durchgeführt wird, entweder einem Disposable oder einem integrierten Disposable, mit der von der Probenentnahmestelle aufzunehmenden, sich im Bereich der Körperteilanlageöffnung 13 befindenden Probe manuell in Kontakt gebracht wird.

### Bezugszeichenliste

- 1: Analysegerät
- 2: Stechgerät
- 3: Fingerkuppe
- 4: Andruckteil
- 5: Antrieb
- 6: Stechelement
- 7: Kapillarkanal
- 8: Analysezone
- 9: Knopf
- 10: Austrittsöffnung
- 11: Bluttropfen
- 12: Beleuchtung
- 13: Körperteilanlageöffnung
- L: Einstechtiefe
- d: Einstechtiefenzugabe

## Patentansprüche

1. Stechgerät (2) zum Erzeugen einer Einstichwunde in der Haut eines Menschen oder Tieres zum Gewinnen einer Probe einer Körperflüssigkeit mit einem ersten analytischen Verbrauchsmittel, das ein Stechelement (6) umfasst, zum Durchführen einer Analyse eines medizinisch bedeutsamen Bestandteils der Probe mit einem zweiten analytischen Verbrauchsmittel, das ein Testelement umfasst, wobei das Stechgerät (2)
ein Andruckteil (4) zum Anpressen des Stechgeräts (2) an einen Körperteil, in dem mit dem Stechelement (6) an einer Probenentnahmestelle eine Einstichwunde erzeugt werden soll, aus der eine Probe entnommen werden soll,
einen Antrieb (5) zum Antreiben eines in das Stechgerät (2) eingesetzten Stechelements (6) für eine Stechbewegung des Stechelements (6) entlang eines vorbestimmten Einstechweges,
ein Gehäuse mit einer Körperteilanlageöffnung (13) für den Körperteil, wobei das Stechelement (6) in einer Ausgangsposition in dem Gehäuse angeordnet ist und in einer Stechposition an der Körperteilanlageöffnung (13) mittels des Antriebs (5) in den angelegten Körperteil einstechbar ist, und
eine Kontrolleinrichtung zum Überprüfen einer gewonnenen Probe daraufhin, ob die gewonnene Probenmenge ausreicht, um mit einem Testelement die Analyse durchzuführen, umfasst,
**dadurch gekennzeichnet, dass**
der Antrieb (5) derart ausgebildet ist, dass mindestens eines der analytischen Verbrauchsmittel in eine Kontaktposition verfahrbar ist, in der es während einer Ausfahrdauer im Bereich der Körperteilanlageöffnung (13) in einer Position verbleibt, in der es von einem Benutzer des Stechgerätes (2) durch eine manuelle Positionierung des Stechgerätes (2) zum manuellen Aufnehmen einer Probe mit der Probenentnahmestelle in Kontakt bringbar ist, wenn gemäß dem Ergebnis einer mit der Kontrolleinrichtung durchgeführten Überprüfung der gewonnenen Probenmenge die bei einer vorausgegangenen Stechbewegung gewonnene Probenmenge nicht zum Durchführen der Analyse oder Diagnose ausreicht.

2. Stechgerät nach Anspruch 1, **dadurch gekennzeichnet, dass** das erste und das zweite analytische Verbrauchsmittel in einem analytischen Verbrauchsmittel integriert sind, das ein integriertes Disposable ist, das sowohl ein Stechelement (6) zum Durchführen des Einstichvorgangs für das Gewinnen der Probe einer Körperflüssigkeit als auch ein Testelement zum Durchführen der Analyse eines medizinisch bedeutsamen Bestandteils der Probe umfasst.

3. Stechgerät (2) nach einem der vorhergehenden Ansprüche, **dadurch gekennzeichnet, dass** die Kontrolleinrichtung zum visuellen Kontrollieren der Probenmenge durch den Benutzer des Gerätes ausgebildet ist.

4. Stechgerät (2) nach einem der vorhergehenden Ansprüche, **dadurch gekennzeichnet, dass** die Kontrolleinrichtung eine automatische Probenmengenerkennungseinrichtung oder Unterdosiererkennungseinrichtung umfasst.

5. Stechgerät (2) nach einem der vorhergehenden Ansprüche, **dadurch gekennzeichnet, dass** die Kontrolleinrichtung eine Signaleinheit zum Signalisieren einer für die Durchführung der Analyse oder Diagnose zu geringen Probenmenge an den Benutzer umfasst.

6. Stechgerät (2) nach einem der vorhergehenden Ansprüche, **dadurch gekennzeichnet, dass** das mindestens eine analytische Verbrauchsmittel mittels des Antriebs (5) in die Kontaktposition verfahrbar ist, wenn bei der Verwendung des Stechelements (6) zum Entnehmen einer Probe erstmals eine für die Durchführung der Analyse oder Diagnose zu geringe Probenmenge erkannt wird.

7. Stechgerät (2) nach den Ansprüchen 4 und 6, **dadurch gekennzeichnet, dass** das mindestens eine analytische Verbrauchsmittel automatisch in die Kontaktposition verfahren wird, wenn die Kontrolleinrichtung eine für die Durchführung der Analyse oder Diagnose zu geringe Probenmenge erkennt.

8. Stechgerät (2) nach einem der vorhergehenden Ansprüche, **dadurch gekennzeichnet, dass** das Stechelement (6) zum Durchführen eines weiteren Einstichvorgangs mit dem Stechelement (6) in die Ausgangs- oder eine Bereitschaftsposition verfahrbar ist, wenn bei der Verwendung des Stechelements (6) zum Entnehmen einer Probe erstmals eine für die Durchführung der Analyse oder Diagnose zu geringe Probenmenge erkannt wird.

9. Stechgerät (2) nach dem vorhergehenden Anspruch, **dadurch gekennzeichnet, dass** der weitere Stechvorgang mit einer vergrößerten Einstichtiefe durchführbar ist.

10. Analysehandgerät (1) zum Analysieren eines medizinisch bedeutsamen Bestandteils einer Körperflüssigkeit, **dadurch gekennzeichnet, dass** es ein Stechgerät (2) nach einem der vorhergehenden Ansprüche umfasst und dass in das Analysegerät (1) eine Analyseeinrichtung zum Durchführen einer Analyse oder Diagnose mittels der von dem Stechgerät (2) gewonnenen Probe integriert ist.

11. Verfahren zum Vorbereiten eines Stechgerätes (2) zum Erzeugen einer Einstichwunde in der Haut eines Menschen oder Tieres zum Gewinnen einer Probe einer Körperflüssigkeit für Analyse- oder Diagnosezwecke mit einem ersten analytischen Verbrauchsmittel, das ein Stechelement (6) umfasst, zum Durchführen einer Analyse eines medizinisch bedeutsamen Bestandteils der Probe mit einem zweiten analytischen Verbrauchsmittel, das ein Testelement umfasst, wobei das Stechgerät (2)
ein Andruckteil (4) zum Anpressen des Stechgeräts (2) an einen Körperteil, in dem mit dem Stechelement (6) an einer Probenentnahmestelle eine Einstichwunde erzeugt werden soll, aus der eine Probe entnommen werden soll,
einen Antrieb (5) zum Antreiben eines in das Stechgerät (2) eingesetzten Stechelements (6) für eine Stechbewegung des Stechelements (6) entlang eines vorbestimmten Einstechweges,
ein Gehäuse mit einer Körperteilanlageöffnung (13) für den Körperteil, wobei das Stechelement (6) in einer Ausgangsposition in dem Gehäuse angeordnet ist und in einer Stechposition an der Körperteilanlageöffnung (13) mittels des Antriebs (5) in den angelegten Körperteil einstechbar ist, und
eine Kontrolleinrichtung zum Überprüfen einer gewonnenen Probe daraufhin, ob die gewonnene Probenmenge ausreicht, um mit einem Testelement die Analyse durchzuführen, umfasst,
**dadurch gekennzeichnet, dass**
mindestens eines der analytischen Verbrauchsmittel mittels des Antriebs (5) in eine Kontaktposition verfahren wird, in der es während einer Ausfahrdauer im Bereich der Körperteilanlageöffnung (13) in einer Position verbleibt, in der es von einem Benutzer des Stechgerätes (2) durch eine manuelle Positionierung des Stechgerätes (2) zum manuellen Aufnehmen einer Probe mit der Probenentnahmestelle in Kontakt bringbar ist, wenn gemäß dem Ergebnis einer mit der Kontrolleinrichtung durchgeführten Überprüfung der gewonnenen Probenmenge die bei einer vorausgegangenen Stechbewegung gewonnene Probenmenge nicht zum Durchführen der Analyse oder Diagnose ausreicht.
